# FASCICULE DE BREVET EUROPEEN

(11) **EP 0 652 209 B1**
(45) Date de publication et mention de la délivrance du brevet: **08.04.1998**
(21) Numéro de dépôt: 94402492.6
(22) Date de dépôt: 04.11.1994
(51) Int. Cl.: C07C 271/22, A61K 7/035, A61K 7/027

(54) **Dérivés de la lysine à groupement N-epsilon-alkoxy ou alcénoxy carbonyle, leur préparation et leur utilisation, dans des compositions cosmétiques, pharmaceutiques, hygiéniques ou alimentaires**
Derivate von N-epsilon Alkoxy- oder Alkenoxy-carbonyl-Lysin, ihre Herstellung und Verwendung in kosmetischen, pharmazeutischen, hygienischen und nahrhaften Zusammensetzungen
Derivatives of N-epsilon alkoxy-or alkenoxy-carbonyl lysine, their preparation and use in cosmetic, pharmaceutical, hygiene or alimentary compositions

(30) Priorité: 05.11.1993 FR 9313188
(43) Date de publication de la demande: 10.05.1995
(73) Titulaire: L'OREAL, 75008 Paris (FR)
(72) Inventeur: Philippe, Michel, F-91320 Wissous (FR); Bordier, Thierry, F-93290 Tremblay-en-France (FR)
(74) Mandataire: Stalla-Bourdillon, Bernard

(56) Documents cités:
- EP-A- 0 139 481
- EP-A- 0 336 265
- EP-A- 0 447 287
- US-A- 3 541 135
- MAKROMOLEKULARE CHEMIE, MACROMOLECULAR CHEMISTRY AND PHYSICS, vol.177, novembre 1976, Basel, CH, pages 3113 - 8, H. GUENIFFEY et al: "Greffage d'acides aminés possédant une troisième fonction sur un polymère, 2"
- SOAP, PERFUMERY AND COSMETICS, vol. 65, no.1, janvier 1992, London, GB, pages 25 - 6, D. WHEELER: "Lipstick, Powder and Patents"

## Description

La présente invention a pour objet de nouveaux dérivés de la lysine à groupement N^{ε} -alkoxy ou alcénoxy carbonyle, leur préparation et leur utilisation, notamment comme agents permettant de faciliter le compactage dans des compositions cosmétiques, pharmaceutiques, hygiéniques ou alimentaires.

Il a été décrit dans EP-139 481, des compositions cosmétiques utilisant comme agents pour modifier la surface de composés inorganiques, en vue d'en augmenter la dispersibilité, soit un dérivé N-mono-acylé d'un acide aminé basique dont le groupement acyle aliphatique a de 8 à 22 atomes de carbone, soit un dérivé N, N-diacylé d'un acide aminé basique dont les groupements acyles aliphatiques, identiques ou différents, ont de 1 à 22 atomes de carbone.

Il a également été décrit dans EP-336 265, des compositions cosmétiques pour la mise en forme des cheveux comprenant comme agents tensioactifs, un dérivé N-monoacylé d'un acide aminé basique dont le groupement acyle aliphatique a de 8 à 22 atomes de carbone.

Les dérivés acylés des acides aminés basiques décrits précédemment sont toutefois très difficilement compactables et même intassables.

Or, on sait que certaines compositions, cosmétiques, pharmaceutiques et hygiéniques sont présentées sous forme dite "compacte". Il s'agit de compositions anhydres constituées principalement de particules solides et d'un liant gras (huiles ou mélange d'huiles et de cires), et mises en forme par compression, ou par coulage dans un conteneur servant de moule.

L'élaboration de telles compositions soulève toutefois de nombreuses difficultés car le produit final doit être suffisamment homogène et compact pour présenter une bonne aptitude au prélèvement et pour éviter par ailleurs une fragmentation pouvant être provoquée notamment par des chocs.

On a maintenant constaté de façon surprenante et inattendue qu'une nouvelle famille de dérivés de la lysine à groupement N^{ε}-alkoxy ou alcénoxy carbonyle permettait non seulement de satisfaire aux exigences précitées et de faciliter l'obtention de telles compositions contrairement aux dérivés N^{ε}-acylés correspondants, mais également de conférer à toute composition cosmétique les contenant des qualités d'étalement, d'adhésion à la peau, et de diffusion de la lumière particulièrement intéressantes, ainsi qu'un toucher onctueux et agréable.

La présente invention a donc pour objet des dérivés de la lysine à groupement N^{ε} -alkoxy ou alcénoxy carbonyle de formule : 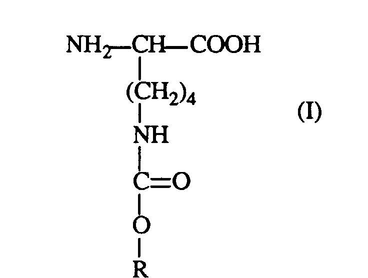 dans laquelle :
R représente un radical alkyle, linéaire ou ramifié, en C₁₃-C₂₄, le radical en C₁₆ étant ramifié, ou un radical alcényle, linéaire ou ramifié en C₈-C₂₄, et les sels desdits composés de formule (I) ainsi que leurs isomères optiques de configuration D ou L ou leurs mélanges.

Parmi les sels des composés de formule (I), on peut citer les sels de cations inorganiques monovalents tels que ceux de sodium, ou divalents tels que ceux de zinc ou de cuivre, et les sels de cations organiques tels que ceux d'aminopropanediol, de trishydroxyaminométhane, de glucamine et de N-méthylglucamine.

Parmi les composés de formule (I), on peut citer en particulier la N^{ε}-hexyl-2-décyloxycarbonyl-L-lysine, la N^{ε}-décyl-2-tétradécyloxycarbonyl-L-lysine et la N^{ε}-tétradécyloxycarbonyl-L-lysine.

Les composés selon l'invention se présentent sous forme solide ayant une taille de particules comprise entre 10 nm et 500 µm et de préférence entre 0,1 et 25 µm.

Ils sont à la fois peu solubles dans les huiles et dans les solutions aqueuses dont le pH est compris entre 5 et 8.

Ils présentent un point de fusion élevé, c'est à dire supérieur à 250°C, un fort pouvoir de réflexion de la lumière, ainsi qu'une excellente adhérence sur la peau.

L'invention a également pour objet un procédé de préparation des composés de formule (I).

Ce procédé consiste à faire réagir en milieu aqueux et à pH basique, de la lysine ou l'un de ses sels, de configuration connue, avec une solution d'un sel de cuivre, puis à faire réagir sur la solution du complexe de cuivre ainsi obtenu de formule : 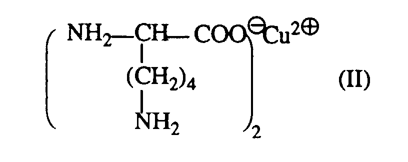 un composé de formule : 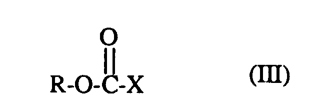 dans laquelle :
R est tel que défini précédemment,
et X est choisi dans le groupe constitué par un atome de chlore, un radical chlorométhyle ou un radical imidazolyle, ledit composé de formule (III) étant additionné sans solvant et à traiter le sel de cuivre de la lysine N^{ε} substituée obtenu par un agent décomplexant et, éventuellement à purifier le composé obtenu.

Parmi les solutions de sels de cuivre utilisées dans le procédé selon l'invention, on peut citer en particulier les solutions de sulfate de cuivre.

Le pH basique du milieu réactionnel, est de préférence compris entre 8 et 14.

Selon un mode de réalisation préféré du procédé de préparation des composés de formule (I) selon l'invention, l'agent décomplexant utilisé est une solution aqueuse du sel disodique de l'acide éthylènediamine-tétracétique.

La présente invention a également pour objet des compositions cosmétiques, pharmaceutiques, hygiéniques ou alimentaires comprenant un dérivé de la lysine correspondant à la formule suivante : 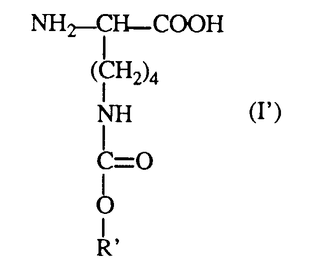 dans laquelle :
R' représente un radical alkyle ou alcényle, linéaire ou ramifié en C₈-C₂₄, et les sels desdits composés de formule (I') ainsi que leurs isomères optiques de configuration D ou L ou leurs mélanges.

Parmi les composés de formule (I') on peut notamment citer la N^{ε}-éthyl-2-hexyloxycarbonyl-L-lysine, la N^{ε}-dodécyloxycarbonyl-L-lysine, la N^{ε}-hexadécyloxycarbonyl-L-lysine, la N^{ε}-décyloxycarbonyl-L-lysine, la N^{ε}- butyl-2-octyloxycarbonyl-L-lysine, la N^{ε}-hexyl-2-décyloxycarbonyl-L-lysine, la N^{ε}-décyl-2-tétradécyloxycarbonyl-L-lysine et la N^{ε}-tétradécyloxycarbonyl-L-lysine.

Certains des composés de formule (I') sont connus et ont été décrits dans US-3541135 comme intermédiaires de composés actifs dans le traitement des allergies et inflammations.

La proportion en dérivé de la lysine de formule (I') dans les compositions selon l'invention est de préférence comprise entre 0,05 % et 80 % en poids par rapport au poids total de la composition et en particulier comprise entre 1 % et 30 % en poids.

Bien entendu, les dérivés de la lysine selon l'invention peuvent être présents dans les compositions selon l'invention sous forme libre ou sous la forme d'une association avec les particules substrats qu'ils enrobent comme mentionné ci-après.

Les compositions selon l'invention peuvent se présenter sous diverses formes telles que des dispersions, des lotions éventuellement épaissies ou gélifiées, des poudres éventuellement "compactées", des laits, des crèmes, des sticks, ou encore des mousses ou des sprays lorsqu'elles sont conditionnées en aérosol.

Elles peuvent être notamment des émulsions huile-dans-eau ou eau-dans-huile, des dispersions vésiculaires ou encore des préparations solides.

Les compositions selon l'invention peuvent notamment se présenter sous forme de compositions cosmétiques pour le maquillage telles que des fonds de teint, des crèmes teintées, des mascaras, des fards à joues et à paupières, des rouges à lèvres, des vernis à ongles et des compositions exfolientes.

Selon une forme particulière de réalisation des compositions selon l'invention, celles-ci se présentent sous forme dite "compacte", les composés de formule générale (I') facilitant le compactage des ingrédients desdites compositions.

Parmi ces compositions dites compactes, on peut citer notamment des fonds de teint, des fards à joues ou à paupières, et les rouges à lèvres.

Les compositions peuvent être également des compositions pharmaceutiques ou hygiéniques telles que des pâtes dentifrices, des poudres pour le corps ou pour bébés et des poudres antitranspirantes.

Outre les composés de formule (I'), les compositions selon l'invention peuvent comprendre au moins un additif choisi dans le groupe constitué par les agents tensio-actifs, les corps gras, les solvants organiques, les silicones, les épaississants, les adoucissants, les filtres solaires, les agents de traitement, les agents anti-mousse, les agents hydratants, les parfums, les conservateurs, les agents antioxydants, les séquestrants, les agents de sapidité, les agents alcalinisants ou acidifiants, les charges, et les poudres minérales ou organiques.

Parmi les agents tensio-actifs utilisables dans les compositions selon l'invention se présentant sous forme d'émulsion, on peut citer les agents tensio-actifs conventionnels qu'ils soient de nature anionique, cationique, non ionique ou amphotère ou un mélange desdits agents tensio-actifs.

Parmi les corps gras utilisables dans les compositions selon l'invention, on peut citer les huiles, les cires, les acides gras, les alcools gras ou un mélange desdits corps gras.

Les huiles peuvent être d'origine animale, végétale, minérale ou de synthèse. On peut citer en particulier l'huile de palme hydrogénée, l'huile de ricin hydrogénée, l'huile de vaseline, l'huile de paraffine et l'huile de purcellin.

Les cires peuvent être d'origine animale, végétale, minérale ou de synthèse. On peut citer en particulier la cire d'abeilles, la cire de montan, la cire de carnauba, la cire de candelilla, la cire de canne à sucre, la cire du Japon, l'ozokérite, les cires microcristallines, la cire de paraffine, la cire de lanoline, la cire de lanoline hydrogénée et la cire de lanoline acétylée.

Parmi les charges utilisables dans les compositions selon l'invention, on peut citer notamment des charges insolubles éventuellement colorées telles que des pigments, par exemple des oxydes métalliques comme les oxydes de titane, de zinc, de fer, de manganèse, de cérium et/ou de zirconium et leurs nanopigments, des poudres de nylon, de polyéthylène, de mica ou de talc.

La présente invention a également pour objet l'utilisation des dérivés de la lysine de formule (I') comme substance de revêtement de particules substrats permettant de conférer auxdites particules de meilleures propriétés, en particulier d'en augmenter la compressibilité et d'améliorer le toucher des compositions cosmétiques les contenant.

Parmi les particules pouvant être ainsi enrobées, on peut citer notamment des pigments, et des charges particulaires tels que mentionnés ci-dessus et des microsphères telles que les microsphères creuses de copolymère de chlorure de vinylidène/acrylonitrile commercialisées sous les dénominations d'"Expancel 551 DE 20" et d'"Expancel 551 DE" par la Société Casco Nobel.

On va maintenant donner à titre d'illustration des exemples de préparation des dérivés de la lysine à groupement N^{ε}-alkoxy ou alcénoxy carbonyle ainsi que de diverses compositions les contenant.

### EXEMPLES DE PREPARATION

### EXEMPLE 1 : Préparation de Nε-dodécyloxycarbonyl)-L-lysine

Dans un tricol de 250 ml muni d'un thermomètre et d'une ampoule d'introduction de 50 ml, on dissout à température ambiante 15 g (82,1 mmoles) de monochlorohydrate de L-lysine dans 66 ml d'une solution aqueuse de soude à 10 %.

On ajoute alors 10,2 g (41,06 mmoles) de sulfate de cuivre pentahydrate préalablement mis en solution dans 30 ml d'eau. Après homogénéisation, on refroidit le milieu réactionnel à une température de 5°C.

On ajoute alors 8,2 g (82,1 mmoles) d'hydrogénocarbonate de sodium, puis goutte à goutte 19,3 g (82,1 mmoles) de chloroformiate de dodécyle. Après une nuit à température ambiante, le milieu réactionnel est filtré et le précipité bleu ainsi obtenu est lavé à l'eau puis, séché sur du pentaoxyde de diphosphore. On traite alors le produit brut sous forme de complexe de cuivre bleu par une solution aqueuse de sel disodique dihydraté de l'acide éthylènediamine-tétracétique à 10 % (120 mmoles) au reflux pendant quatre heures, puis on refroidit le mélange, on essore le précipité, le lave à l'eau et à l'acétone, puis le sèche.

Ce traitement est ensuite répété jusqu'à obtention d'un produit blanc.

On obtient ainsi, avec un rendement de 74 %, 21,8 g de N^{ε}-dodécyloxycarbonyl-L-lysine se présentant sous forme d'une poudre blanche.

### Analyses :

Point de fusion: > 260°C (Kofler)
Spectre de masse : (TSQ 70 CI-DCI) : m/Z: 359(M+H) ; 315(M-CO₂+H)⁺; 169(C₁₂H₂₅⁺)
Les spectres RMN sont enregistrés sur un Bruker WM 250
¹H RMN (CD₃COOD) :
4,12 ppm (3H, m, CH₂-12 et CH-18) ;
3,22 ppm (2H, t, CH₂-14) ;
1,26 à 1,67 ppm (26 H, m, CH₂-2 à 11 et 15 à 17) ;
0,95 ppm (3H, t, CH₃ -1)

| Analyse élémentaire C₁₉H₃₈N₂O₄ ; PM = 358,526 | | | | |
|---|---|---|---|---|
| | C % | H % | N % | O % |
| Calculé | 63,65 | 10,68 | 7,81 | 17,85 |
| Trouvé | 63,43 | 10,65 | 8,05 | 17,88 |

Présence de cuivre : 695 ppm
Granulométrie mesurée à l'aide d'un Coulter counter TA II : Taille moyenne (en nombre) = 2,23 µm
Ecart type = 1,36 µm.

### EXEMPLE 2 : Préparation de N^{ε} -hexadécyloxycarbonyl-L-lysine

Selon le même mode opératoire que décrit dans l'exemple 1, on obtient en utilisant 25 g (82,1 mmoles) de chloroformiate d'hexadécyle, 29,1 g de N^{ε}-hexadécyloxycarbonyl-L-lysine (Rendement 85,5 %) se présentant sous forme d'une poudre blanche.

### Analyses :

Point de fusion: > 260°C (Kofler)
Spectre de masse : (TSQ 70 CI-DCI) : m/Z : 415(MH); 367,2 ; 279,1; 225,1

| Analyse élémentaire C₂₃H₄₆N₂O₄ ; PM = 414,634 | | | | |
|---|---|---|---|---|
| | C % | H % | N % | O % |
| Calculé | 66,56 | 11,09 | 6,75 | 15,43 |
| Trouvé | 66,70 | 11,07 | 6,58 | 15,18 |

Présence de cuivre : 0,45 %
Granulométrie mesurée à l'aide d'un Coulter counter TA II : Taille moyenne (en nombre) = 4,58 µm
Ecart type = 5,62 µm.

### EXEMPLE 3 : Préparation de N^{ε} -éthyl-2-hexyloxycarbonyl-L-lysine

Selon le même mode opératoire que décrit dans l'exemple 1, on obtient, en utilisant 14,8 g (82,1 mmoles) de chloroformiate d'éthyl-2-hexyle, 8,4 g de N^{ε}-éthyl-2-hexyloxycarbonyl-L-lysine (Rendement 34 %) se présentant sous forme d'une poudre blanche.

### Analyses :

Point de fusion : > 260°C (Kofler)
Les spectres RMN sont enregistrés sur un Bruker WM 250
¹H RMN (CD₃COOD) :
4,07 à 4,15 ppm (3H, t+m, CH₂-8 CH-14 ;
3,23 ppm (2H, t, CH₂-10) ;
1,98 à 2,1 ppm (2H, m, CH₂-13);
1,38 à 1,61 ppm (13H, m, CH₂-2 à 4 et 6,11,12 et CH-5) ;
0,97 ppm (6H, 2t, CH₃-1 et 7)

| Analyse élémentaire C₁₅H₃₀N₂O₄ ; PM = 302,417 | | | | |
|---|---|---|---|---|
| | C % | H % | N % | O % |
| Calculé | 59,58 | 10,00 | 9,26 | 21,16 |
| Trouvé | 58,83 | 10,06 | 9,13 | 21,65 |

Présence de cuivre : 100 ppm
Granulométrie mesurée à l'aide d'un Coulter counter TA II : Taille moyenne (en nombre) : 2,57 µm ;
Ecart type = 1,86 µm.

### EXEMPLE 4 : N^{ε}-décyloxycarbonyl-L-lysine

Selon le même mode opératoire que décrit dans l'exemple 1, en utilisant comme chloroformiate d'alkyle le chloroformiate de décyle, on obtient la N^{ε}-décyloxycarbonyl-L-lysine se présentant sous forme d'une poudre blanche.

### Analyses :

Point de fusion : 244,9°C (DSC Mettler)
Spectre de masse : (SSQ 710 CI-DI) : m/Z : 331 (MH)⁺
Les spectres RMN sont enregistrés sur un Bruker AMXS
¹H RMN (CD₃COOD) :
4,17 ppm (2H, t, CH₂-10) ;
4,10 ppm (1H, m, CH-16) ;
3,25 ppm (2H, t, CH₂-12) ;
2,06 ppm (2H, m, CH₂-15) ;
1,70 ppm (2H, m, CH₂-9) ;
1,64 ppm (2H, m, CH₂-13) ;
1,40 ppm (16H, m, CH₂-2 à 8 et 14) ;
0,970 ppm (3H, t, CH₃-1).

| Analyse élémentaire C₁₇H₃₄N₂O₄ ; PM = 330,5. | | | | |
|---|---|---|---|---|
| | C % | H % | N % | O % |
| Calculé | 61,72 | 10,29 | 8,47 | 19,36 |
| Trouvé | 61,97 | 10,50 | 8,41 | 19,43 |

Présence de cuivre : 340 ppm.

Granulométrie mesurée par diffraction de la lumière à l'aide d'un Leeds & Worthrup, modèle Microtrac X100 à 0,4 % dans un mélange eau/éthanol (1/1) : Taille moyenne (en nombre) : 0,69 µm.

### EXEMPLE 5 : N^{ε}-butyl-2-octyloxycarbonyl-L-lysine

Selon le même mode opératoire que décrit dans l'exemple 1, en utilisant comme chloroformiate d'alkyle le chloroformiate de butyl-2-octyle, on obtient la N^{ε}-butyl-2-octyloxycarbonyl-L-lysine se présentant sous forme d'une poudre blanche.

### Analyses :

Point de fusion : ≥ 260°C (Baukofler)
Spectre de masse : (SSQ 710 CI-DI) : m/Z : 359 (MH)⁺
Les spectres RMN sont enregistrés sur un Bruker AMX 500
¹H RMN (DMSO d₆ + CD₃COOD) :
3,81 ppm (2H, d, CH₂-12) ;
3,52 ppm (1H, dd, CH-18) ;
2,94 ppm (2H, t, CH₂-14) ;
1,66 à 1,77 ppm (2H, 2m, CH₂-17) ;
1,51 ppm (1H, m, CH-7) ;
1,21 à 1,40 (20H, m, CH₂-2 à 6,8 à 10 et 15,16) ;
0,82 ppm (6H, 2t, CH₃-1 et 11).

| Analyse élémentaire C₁₉H₃₈N₂O₄ ; PM = 358,526 | | | | |
|---|---|---|---|---|
| | C % | H % | N % | O % |
| Calculé | 63,65 | 10,68 | 7,81 | 17,85 |
| Trouvé | 63,78 | 10,69 | 7,75 | 18,14 |

Présence de cuivre : 60 ppm.

Granulométrie mesurée par diffraction de la lumière à l'aide d'un Leeds & Worthrup modèle Microtrac X100 à 0,4 % dans un mélange eau/éthanol (1/1) : Taille moyenne (en nombre) : 1,66 µm.

### EXEMPLE 6 : N^{ε}-dodécyloxycarbonyl-L-lysinate de zinc

Dans un bécher de 250 ml, on dissout à 70°C, 5 g (13,9 mmoles) de N^{ε}-dodécyloxycarbonyl-L-lysine obtenue à l'exemple 1 dans 5,6 ml d'une solution aqueuse de soude à 10 % et 50 ml d'eau.

On ajoute alors goutte à goutte 2 g de sulfate de zinc heptahydrate préalablement mis en solution dans 10 ml d'eau.

Le milieu réactionnel est filtré et le précipité blanc ainsi obtenu est lavé à l'eau puis séché.

On obtient ainsi, avec un rendement de 96 %, 5,2 g de N^{ε}-dodécyloxycarbonyl-L-lysinate de zinc se présentant sous forme d'une poudre blanche.

### Analyses :

Point de fusion : > 260°C (Baukofler)

| Analyse élémentaire : 2C₁₉H₃₇N₂O₄-Zn ; PM = 780,405 | | | | | |
|---|---|---|---|---|---|
| | C % | H % | N % | O % | Zn % |
| Calculé | 58,49 | 9,56 | 7,18 | 16,4 | 8,38 |
| %Calc. (+ 1,2 moles H₂O) | 56,85 | 9,52 | 6,98 | 18,35 | 8,15 |
| Trouvé | 56,88 | 9,25 | 7,09 | 18,91 | 8,00 |

Granulométrie mesurée par diffraction de la lumière à l'aide d'un Leeds & Worthrup modèle Microtrac X 100 à 0,4 % dans un mélange eau/éthanol (1/1) : Taille moyenne (en nombre) : 0,85 µm.

### EXEMPLES DE COMPOSITION

### EXEMPLE I : Poudre compacte

On prépare séparément les phases A et B par mélange des ingrédients suivants :

### Phase A :

- Talc 36,7 g
- Oxychlorure de bismuth 10 g
- Stéarate de zinc 4 g
- N^{ε}-dodécyloxycarbonyl-L-lysine 20 g
- Poudre de Nylon 12 commercialisée sous la dénomination de "Orgasol 2082 D Nat Extra Cos" par la Société Atochem 20 g
- Parfum 1,6 g
- Microsphères creuses commercialisées sous la dénomination de "Expancel 551 DE 20" par la Société Casco Nobel 0,1 g
- Oxyde de fer jaune commercialisé sous la dénomination de "Sicomet Jaune 10" par la Société BASF 0,6 g
- Mélange d'oxyde de fer brun et d'oxyde de fer jaune commercialisé sous la dénomination de "Sicomet Brun ZP 3569" par la Société BASF 0,8 g
- Oxyde de fer noir commercialisé sous la dénomination de "Sicomet noir 85" par la Société BASF 0,2 g

### Phase B :

- Huile de vaseline 6 g

On broie doucement l'ensemble des ingrédients de la phase A pendant 5 minutes puis ajoute la phase B. On broie alors le mélange doucement pendant 2 minutes puis vivement pendant 3 minutes.

On tamise ensuite la composition à l'aide d'un tamis de 0,160 mm, et on répartit la préparation dans des coupelles puis compacte à une pression de 60 bars.

La poudre compacte ainsi obtenue présente une dureté satisfaisante, résistant bien aux chocs.

Elle se délite aisément et s'étale facilement sur la peau en présentant une adhérence très satisfaisante.

### EXEMPLE II : Rouges à lèvres

On procède au mélange des ingrédients suivants :
- Huile de jojoba 11,65 g
- Huile de sésame 11,31 g
- Di-tertio-butyl-hydroxy-4 toluène commercialisé sous la dénomination de "Antracine 8" par la Société Jan Dekker 0,07 g
- Lanolate d'isopropyle stabilisé commercialisé sous la dénomination de "Lanesta SLPV" par la Société Westbrook 12,48 g
- Béhénate d'éthyl-2-hexyl glycéryle commercialisé sous la dénomination de "Mexanyl GQ" par la Société Chimex 11,31 g
- Polybutylène commercialisé sous la dénomination de "Indopol H 300" par la Société Amoco Chemical 12,48 g

On chauffe le mélange au bain marie pendant une heure à température comprise entre 60 et 70°C, puis on ajoute le mélange des ingrédients suivants :
- Laque de calcium du Rouge Lithol B sur colophane commercialisée sous la dénomination de "DC Red 7 WOO5" par la Société Wackherr 2,1 g
- Laque d'aluminium du Jaune Brillant FCF sur alumine commercialisée sous la dénomination de "FDC Yellow 6 Al Lake BC - 6508" par la Société Clark Color 7,32 g
- Oxyde de fer jaune 0,2 g
- Mélange d'oxyde de fer brun et d'oxyde de fer jaune commercialisé sous la dénomination de "Sicomet brun ZP 3569" par la Société BASF 1,35 g
- Oxyde de titane rutile non traité commercialisé sous la dénomination de "Hombitan R 301" par la Société Sachtleben 0,649 g

On broie le mélange ainsi obtenu à l'aide d'un broyeur Discontimill, puis on ajoute le mélange des ingrédients suivants :
- Cire d'abeille 3,18 g
- Cire de polyéthylène commercialisée sous la dénomination de "Polywax 500" par la Société Bareco 12,74 g
- Cire de lanoline oxypropylénée commercialisée sous la dénomination de "Propoxyol 1695" par la Société Henkel 12,86 g

Après chauffage du mélange pendant une heure à 100°C, on obtient un mélange homogène auquel on ajoute 0,3 g de parfum. Lorsque la température du mélange est redescendue à environ 60°C, on ajoute 2 g de N^{ε}-dodécyloxycarbonyl-L-lysine, puis on coule le mélange dans des moules.

Les rouges à lèvres ainsi obtenus sont à la fois homogènes, compacts et présentent une bonne résistance aux chocs.

Ils s'appliquent facilement sur les lèvres, sont onctueux et présentent une bonne tenue.

### EXEMPLE III : Crème démaquillante

On procède au mélange des ingrédients suivants :
- Mono-stéarate de sorbitane commercialisé sous la dénomination de "Span 60" par la Société ICI Surfactants 6 g
- Alcool oléylique polyglycérolé commercialisé sous la dénomination de "Chimexane NB" par la Société Chimex 4 g
- Allantoïne 0,2 g
- D-Panthénol 0,8 g
- Eau 78,98 g

Après chauffage du mélange à 80°C, on ajoute sous agitation à l'aide d'un agitateur Moritz, 10 g de N^{ε}-dodécyloxycarbonyl-L-lysine, puis on homogénéise.

Lorsque la température du mélange est redescendue à environ 40°C, on ajoute 0,02 g de conservateur commercialisé sous la dénomination de "Kathon CG" par la Société Rohm-Haas.

La crème ainsi obtenue s'applique facilement sur la peau et est très onctueuse.

### EXEMPLE IV : Rouges à lèvres

Selon le même mode opératoire que décrit à l'exemple II, on prépare des rouges à lèvres à partir des ingrédients suivants :
- Huile de jojoba 11,65 g
- Huile de sésame 11,31 g
- Di-tertio-butyl-hydroxy-4 toluène commercialisé sous la dénomination de "Antracine 8" par la Société Jan Dekker 0,07 g
- Lanolate d'isopropyle stabilisé commercialisé sous la dénomination de "Lanesta SLPV" par la Société Westbrook 12,48 g
- Béhénate d'éthyl-2-hexyl glycéryle commercialisé sous la dénomination de "Mexanyl GQ" par la Société Chimex 11,31 g
- Polybutylène commercialisé sous la dénomination de "Indopol H 300" par la Société Amoco Chemical 12,48 g
- Laque de calcium du Rouge Lithol B sur colophane commercialisée sous la dénomination de "DC Red 7 WOO5" par la Société Wackherr 2,1 g
- Laque d'aluminium du Jaune Brillant FCF sur alumine commercialisée sous la dénomination de "FDC Yellow 6 Al Lake BC - 6508" par la Société Clark Color 7,32 g
- Oxyde de fer jaune 0,2 g
- Mélange d'oxyde de fer brun et d'oxyde de fer jaune commercialisé sous la dénomination de "Sicomet brun ZP 3569" par la Société BASF 1,35 g
- Oxyde de titane rutile non traité commercialisé sous la dénomination de "Hombitan R 301" par la Société Sachtleben 0,649g
- Cire d'abeille 3,18 g
- Cire de polyéthylène commercialisée sous la dénomination de "Polywax 500" par la Société Bareco 12,74 g
- Cire de lanoline oxypropylénée commercialisée sous la dénomination de "Propoxyol 1695" par la Société Henkel 12,86 g
- N^{ε}-butyl-2-octyloxycarbonyl-L-lysine 2,0 g

Les rouges à lèvres ainsi obtenus sont à la fois homogènes, compacts et présentent une bonne résistance aux chocs.

Ils s'appliquent facilement sur les lèvres, sont onctueux et présentent une bonne tenue.

### EXEMPLE V : Rouges à lèvres

Selon le même mode opératoire que décrit à l'exemple II, on prépare des rouges à lèvres à partir des ingrédients suivants :
- Huile de jojoba 11,65 g
- Huile de sésame 11,31 g
- Di-tertio-butyl-hydroxy-4 toluène commercialisé sous la dénomination de "Antracine 8" par la Société Jan Dekker 0,07 g
- Lanolate d'isopropyle stabilisé commercialisé sous la dénomination de "Lanesta SLPV" par la Société Westbrook 12,48 g
- Béhénate d'éthyl-2-hexyl glycéryle commercialisé sous la dénomination de "Mexanyl GQ" par la Société Chimex 11,31 g
- Polybutylène commercialisé sous la dénomination de "Indopol H 300" par la Société Amoco Chemical 12,48 g
- Laque de calcium du Rouge Lithol B sur colophane commercialisée sous la dénomination de "DC Red 7 WOO5" par la Société Wackherr 2,1 g
- Laque d'aluminium du Jaune Brillant FCF sur alumine commercialisée sous la dénomination de "FDC Yellow 6 Al Lake BC - 6508" par la Société Clark Color 7,32 g
- Oxyde de fer jaune 0,2 g
- Mélange d'oxyde de fer brun et d'oxyde de fer jaune commercialisé sous la dénomination de "Sicomet brun ZP 3569" par la Société BASF 1,35 g
- Oxyde de titane rutile non traité commercialisé sous la dénomination de "Hombitan R 301" par la Société Sachtleben 0,649 g
- Cire d'abeille 3,18 g
- Cire de polyéthylène commercialisée sous la dénomination de "Polywax 500" par la Société Bareco 12,74 g
- Cire de lanoline oxypropylénée commercialisée sous la dénomination de "Propoxyol 1695" par la Société Henkel 12,86 g
- N^{ε}-dodécyloxycarbonyl-L-lysinate de zinc 2,0 g

Les rouges à lèvres ainsi obtenus sont à la fois homogènes, compacts et présentent une bonne résistance aux chocs.

Ils s'appliquent facilement sur les lèvres, sont onctueux et présentent une bonne tenue.

## Revendications

1. Dérivés de la lysine à groupement N^{ε}-alkoxy ou alcénoxy carbonyle de formule : 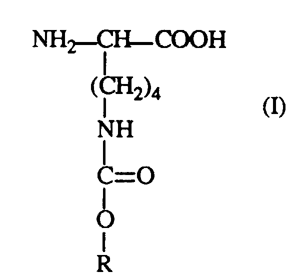 dans laquelle :
R représente un radical alkyle, linéaire ou ramifié, en C₁₃-C₂₄, le radical en C₁₆ étant ramifié, ou un radical alcényle, linéaire ou ramifié, en C₈-C₂₄ et les sels desdits composés de formule (I) ainsi que leurs isomères optiques de configuration D ou L ou leurs mélanges.

2. Dérivés de la lysine selon la revendication 1, caractérisés par le fait que lesdits sels sont des sels de cations inorganiques ou organiques.

3. Dérivés de la lysine selon l'une quelconque des revendications 1 à 2, caractérisés par le fait qu'ils sont choisis dans le groupe constitué par : la N^{ε}-hexyl-2-décyloxycarbonyl-L-lysine, la N^{ε}-décyl-2-tétradécyloxycarbonyl-L-lysine et la N^{ε}-tétradécyloxycarbonyl-L-lysine.

4. Dérivés de la lysine selon l'une quelconque des revendications précédentes caractérisés par le fait que lesdits dérivés présentent un point de fusion supérieur à 250°C et ont une taille de particules comprise entre 10 nm et 500 µm et de préférence entre 0,1 et 25 µm.

5. Procédé de préparation des dérivés de la lysine de formule (I) tel que définis selon l'une quelconque des revendications précédentes, caractérisé par le fait qu'il consiste à faire réagir en milieu aqueux et à pH basique, de la lysine ou l'un de ses sels, de configuration connue, avec une solution d'un sel de cuivre, puis à faire réagir sur la solution du complexe de cuivre ainsi obtenu, de formule : 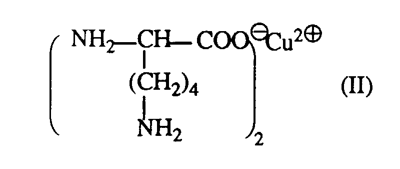 un composé de formule : 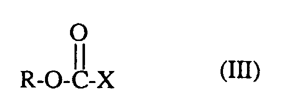 dans laquelle :
R est tel que défini selon la revendication 1,
et X est choisi dans le groupe constitué par un atome de chlore, un radical chlorométhyle et un radical imidazolyle, ledit composé de formule (III) étant additionné sans solvant, et à traiter le sel de cuivre de la lysine N^{ε}- substituée ainsi obtenu par un agent décomplexant et, à purifier éventuellement le composé obtenu.

6. Procédé de préparation selon le revendication 5, caractérisé par le fait que ladite solution de sel de cuivre est une solution de sulfate de cuivre.

7. Procédé de préparation selon l'une quelconque des revendications 5 ou 6, caractérisé par le fait que l'agent décomplexant est une solution aqueuse de sel disodique de l'acide éthylènediaminetétracétique.

8. Composition cosmétique, pharmaceutique, hygiénique ou alimentaire, caractérisée par le fait qu'elle contient un dérivé de la lysine correspondant à la formule suivante : 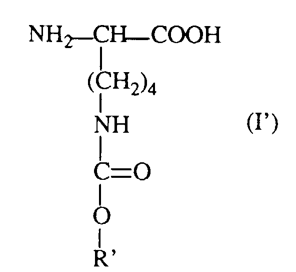 dans laquelle :
R' représente un radical alkyle ou alcényle, linéaire ou ramifié en C₈-C₂₄, et les sels desdits composés de formule (I') ainsi que leurs isomères optiques de configuration D ou L ou leurs mélanges.

9. Composition selon la revendication 8, caractérisée par le fait que le dérivé de la lysine de formule (I') est choisi dans le groupe constitué par la N^{ε}-éthyl-2-hexyloxycarbonyl-L-lysine, la N^{ε}-dodécyloxycarbonyl-L-lysine, la N^{ε}-hexadécyloxycarbonyl-L-lysine, la N^{ε}-décyloxycarbonyl-L-lysine, la N^{ε}- butyl-2-octyloxycarbonyl-L-lysine, la N^{ε}-hexyl-2-décyloxycarbonyl-L-lysine, la N^{ε}-décyl-2-tétradécyloxycarbonyl-L-lysine et la N^{ε}-tétradécyloxycarbonyl-L-lysine.

10. Composition selon la revendication 8 ou 9, caractérisée par le fait que ledit dérivé de la lysine est présent en une proportion comprise entre 0,05 % et 80 % en poids par rapport au poids total de la composition.

11. Composition selon l'une quelconque des revendications 8 à 10, caractérisée par le fait que ledit dérivé de la lysine est présent en une proportion comprise entre 1 % et 30 % en poids par rapport au poids total de la composition.

12. Composition selon l'une quelconque des revendications 8 à 11, caractérisée par le fait qu'elle contient en outre au moins un additif choisi dans le groupe constitué par les agents tensio-actifs, les corps gras, les solvants organiques, les silicones, les épaississants, les adoucissants, les filtres solaires, les agents de traitement, les agents anti-mousse, les agents hydratants, les parfums, les conservateurs, les agents anti-oxydants, les séquestrants, les agents de sapidité, les agents alcalinisants ou acidifiants, les charges, et les poudres minérales ou organiques.

13. Utilisation d'un dérivé de la lysine correspondant à la formule (I') selon les revendications 8 et 9, comme substance de revêtement de particules substrats.

14. Utilisation d'un dérivé de la lysine selon la revendication 13, caractérisée par le fait que les particules substrats sont choisies parmi les charges insolubles éventuellement colorées.

15. Utilisation d'un dérivé de la lysine selon la revendication 14, caractérisée par le fait que lesdites charges sont choisies parmi les oxydes métalliques de zinc, de fer, de titane, de manganèse, de cérium et/ou de zirconium, et leurs nanopigments ainsi que les poudres de nylon, de polyéthylène, de mica ou de talc.

## Claims

1. Lysine derivatives containing an N^{ε}-alkoxy or N^{ε}-alkenoxycarbonyl group of formula: 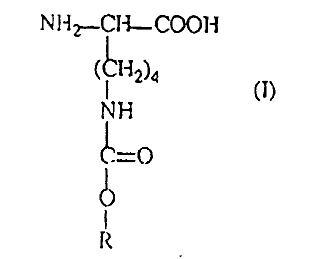 in which:
R represents a linear or branched C₁₃-C₂₄ alkyl radical, the C₁₆ radical being branched, or a linear or branched C₈-C₂₄ alkenyl radical, and the salts of the said compounds of formula (I) as well as their optical isomers of D or L configuration or their mixtures.

2. Lysine derivatives according to Claim 1, characterized in that the said salts are salts of inorganic or organic cations.

3. Lysine derivatives according to either of Claims 1 and 2, characterized in that they are chosen from the group consisting of N^{ε}-2-hexyldecyloxycarbonyl-L-lysine, N^{ε}-2-decyltetradecyloxycarbonyl-L-lysine and N^{ε}-tetradecyloxycarbonyl-L-lysine.

4. Lysine derivatives according to any one of the preceding claims, characterized in that the said derivatives have a melting point greater than 250°C and have a particle size of between 10 nm and 500 µm and preferably between 0.1 and 25 µm.

5. Process for the preparation of the lysine derivatives of formula (I) as defined according to any one of the preceding claims, characterized in that it consists in reacting lysine or one of its salts, of known configuration, in aqueous medium and at basic pH, with a solution of a copper salt, in then reacting the solution of the copper complex thus obtained, of formula: 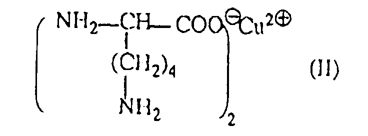 with a compound of formula: 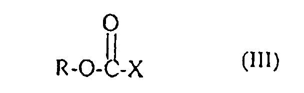 in which:
R is as defined according to Claim 1,
and X is chosen from the group consisting of a chlorine atom, a chloromethyl radical and an imidazolyl radical, the said compound of formula (III) being added without solvent, and
in treating the copper salt of the N^{ε}-substituted lysine thus obtained with a decomplexing agent
and, optionally, in purifying the compound obtained.

6. Preparation process according to Claim 5, characterized in that the said copper salt solution is a copper sulphate solution.

7. Preparation process according to either of Claims 5 and 6, characterized in that the decomplexing agent is an aqueous solution of the disodium salt of ethylenediaminetetraacetic acid.

8. Cosmetic, pharmaceutical, hygiene or food composition, characterized in that it contains a lysine derivative corresponding to the following formula: 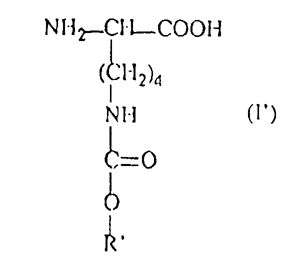 in which:
R' represents a linear or branched C₈-C₂₄ alkyl or alkenyl radical, and the salts of the said compounds of formula (I') as well as their optical isomers of D or L configuration or their mixtures.

9. Composition according to Claim 8, characterized in that the lysine derivative of formula (I') is chosen from the group consisting of N^{ε}-2-ethylhexyloxycarbonyl-L-lysine, N^{ε}-dodecyloxycarbonyl-L-lysine, N^{ε} -hexadecyloxycarbonyl-L-lysine, N^{ε}-decyloxycarbonyl-L-lysine, N^{ε}-2-butyloctyloxycarbonyl-L-lysine, N^{ε}-2-hexyldecyloxycarbonyl-L-lysine, N^{ε} -2-decyltetradecyloxycarbonyl-L-lysine and N^{ε} -tetradecyloxycarbonyl-L-lysine.

10. Composition according to Claim 8 or 9, characterized in that the said lysine derivative is present in a proportion of between 0.05% and 80% by weight with respect to the total weight of the composition.

11. Composition according to any one of Claims 8 to 10, characterized in that the said lysine derivative is present in a proportion of between 1% and 30% by weight with respect to the total weight of the composition.

12. Composition according to any one of Claims 8 to 11, characterized in that it additionally contains at least one additive chosen from the group consisting of surface-active agents, fatty substances, organic solvents, silicones, thickeners, emollients, sunscreening agents, treating agents, anti-foaming agents, moisturizing agents, fragrances, preservatives, anti-oxidizing agents, sequestrants, flavouring agents, basifying or acidifying agents, fillers and inorganic or organic powders.

13. Use of a lysine derivative corresponding to the formula (I') according to Claims 8 and 9 as substance for coating substrate particles.

14. Use of a lysine derivative according to Claim 13, characterized in that the substrate particles are chosen from optionally coloured insoluble fillers.

15. Use of a lysine derivative according to Claim 14, characterized in that the said fillers are chosen from metal oxides of zinc, iron, titanium, manganese, cerium and/or zirconium, and their nanopigments, or nylon, polyethylene, mica or talc powders.

## Patentansprüche

1. Lysinderivate mit der Gruppierung N^{ε}-Alkoxy- oder -Alkenoxycarbonyl der folgenden Formel: 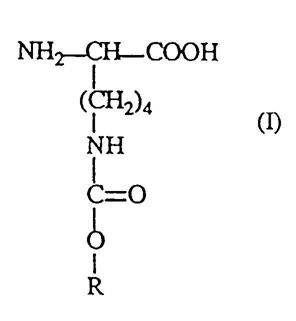 worin:
R einen gerad- oder verzweigtkettigen C₁₃-C₂₄-Alkylrest bedeutet, wobei der C₁₆-Rest verzweigt ist, oder einen gerad- oder verzweigtkettigen C₈-C₂₄-Alkenylrest darstellt sowie
die Salze der Verbindungen gemäß der Formel (I) wie auch deren optische Isomere mit der D- oder L-Konfiguration bzw. deren Gemischen.

2. Lysinderivate nach Anspruch 1, dadurch gekennzeichnet, daß die Salze anorganische oder organische Kationensalze darstellen.

3. Lysinderivate nach einem der Ansprüche 1 bis 2, dadurch gekennzeichnet, daß sie aus der aus N^{ε}-Hexyl-2-decyloxycarbonyl-L-lysin, N^{ε}-Decyl-2-tetradecyloxycarbonyl-L-lysin und dem N^{ε}-Tetradecyloxycarbonyl-L-lysin bestehenden Gruppe ausgewählt sind.

4. Lysinderivate nach einem der vorstehenden Ansprüche, dadurch gekennzeichnet, daß die Derivate einen Schmelzpunkt von höher als 250 °C und eine Teilchengröße aufweisen, die zwischen 10 nm und 500 µm, vorzugsweise zwischen 0,1 und 25 µm beträgt.

5. Verfahren zur Herstellung von Lysinderivaten der Formel (I) gemäß der in einem der vorstehenden Ansprüche gegebenen Bedeutung, dadurch gekennzeichnet, daß es darin besteht, in einem wäßrigen Milieu und bei einem basischen pH-Wert Lysin oder eines seiner Salze bekannter Konfiguration mit einer Lösung eines Kupfersalzes zur Reaktion zu bringen, und anschließend die Lösung des so erhaltenen Kupferkomplexes der Formel 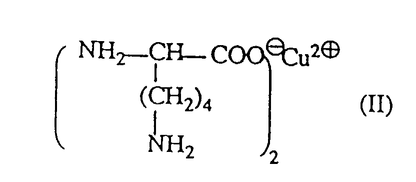 mit einer Verbindung der Formel 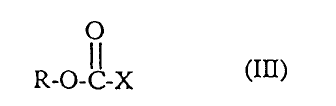 reagieren zu lassen,
worin:
R die in Anspruch 1 gegebene Bedeutung aufweist, und
X aus der aus einem Chloratom, einer Chlormethylgruppe und einem Imidazolylrest bestehenden Gruppe ausgewählt wird, wobei die Verbindung gemäß der Formel (III) ohne Lösungsmittel hinzugefügt wird, und die Behandlung des so erhaltenen Kupfersalzes des N^{ε}-substituierten Lysins mit einem Dekomplexierungsmittel und gegebenenfalls die Reinigung der erhaltenen Verbindung erfolgen.

6. Verfahren zur Herstellung nach Anspruch 5, dadurch gekennzeichnet, daß die Lösung des Kupfersalzes eine Kupfersulfatlösung darstellt.

7. Verfahren zur Herstellung nach einem der Ansprüche 5 oder 6, dadurch gekennzeichnet, daß das Dekomplexierungsmittel eine wäßrige Lösung aus dem Dinatriumsalz der Ethylendiamintetraessigsäure darstellt.

8. Kosmetische, pharmazeutische, hygienische oder Nahrungsmittelzubereitung, dadurch gekennzeichnet, daß sie ein Lysinderivat gemäß der folgenden Formel enthält: 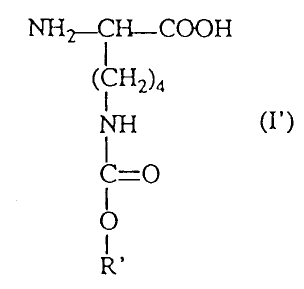 worin:
R' einen gerad- oder verzweigtkettigen C₈-C₂₄-Alkyl- oder -Alkenylrest bedeutet, sowie
die Salze der Verbindungen gemäß der Formel (I') wie auch deren optische Isomere der Konfiguration D oder L oder deren Gemische.

9. Zubereitung nach Anspruch 8, dadurch gekennzeichnet, daß das Lysinderivat gemäß der Formel (I') aus der aus N^{ε}-Ethyl-2-hexyloxycarbonyl-L-lysin, N^{ε}-Dodecyloxycarbonyl-L-lysin, N^{ε}-Hexadecyloxycarbonyl-L-lysin, N^{ε}-Decyloxycarbonyl-L-lysin, N^{ε} -Butyl-2-octyloxycarbonyl-L-lysin, N^{ε}-Hexyl-2-decyloxycarbonyl-L-lysin, N^{ε}-Decyl-2-tetradecyloxycarbonyl-L-lysin, und N^{ε}-Tetradecyloxycarbonyl-L-lysin bestehenden Gruppe ausgewählt ist.

10. Zubereitung nach Anspruch 8 oder 9, dadurch gekennzeichnet, daß das Lysinderivat in einem Mengenverhältnis im Bereich zwischen 0,05 Gew.-% und 80 Gew.-% in bezug auf das Gesamtgewicht der Zusammensetzung vorhanden ist.

11. Zubereitung nach einem der Ansprüche 8 bis 10, dadurch gekennzeichnet, daß das Lysinderivat in einem Mengenverhältnis im Bereich zwischen 1 Gew.-% und 30 Gew.-% in bezug auf das Gesamtgewicht der Zusammensetzung vorhanden ist.

12. Zubereitung nach einem der Ansprüche 8 bis 11, dadurch gekennzeichnet, daß sie zusätzlich noch mindestens ein Additiv enthält, das aus der aus Tensiden, Fettkörpern, organischen Lösungsmitteln, Silikonen, Verdickungsmitteln, Erweichungsmitteln, Sonnenfiltersubstanzen, Wirkstoffen zur Behandlung, Antischaummitteln, Hydratisierungsmitteln, Duftstoffen, Konservierungsmitteln, Antioxidantien, Komplexbildnern, Mitteln zur Geschmacksverbesserung, Alkalisierungs- oder Ansäuerungsmitteln, Trägerstoffen, mineralischen oder organischen Pudern bestehenden Gruppe ausgewählt ist.

13. Verwendung eines Lysinderivats gemäß der Formel (I') nach den Ansprüchen 8 und 9 als Umhüllungssubstanz von Substratteilchen.

14. Verwendung eines Lysinderivats nach Anspruch 13, dadurch gekennzeichnet, daß die Substratteilchen unter unlöslichen, gegebenenfalls gefärbten Trägern ausgewählt werden.

15. Verwendung eines Lysinderivats nach Anspruch 14, dadurch gekennzeichnet, daß die Trägerstoffe unter Metalloxiden von Zink, Eisen, Titan, Mangan, Cer und/oder Zirkonium, sowie Nanopigmenten wie auch Nylon- oder Polyethylenpudern, Glimmer oder Talkum ausgewählt sind.
